# EUROPEAN PATENT SPECIFICATION

(11) **EP 1 339 412 B1**
(45) Date of publication and mention of the grant of the patent: **02.11.2011**
(21) Application number: 01983307.8
(22) Date of filing: 14.11.2001
(51) Int. Cl.: A61K 31/662, A61K 9/107, A61P 17/16

(54) **FORMULATION CONTAINING PHOSPHATE DERIVATIVES OF ELECTRON TRANSFER AGENTS**
FORMULIERUNG ENTHALTEND PHOSPHATDERIVATE VON ELEKTRONENTRANSFERMITTELN
FORMULATION CONTENANT DES DERIVES DE PHOSPHATE D'AGENTS DE TRANSFERT D'ELECTRONS

(30) Priority: 14.11.2000 US 247997 P; 06.06.2001 AU PR549901
(43) Date of publication of application: 03.09.2003
(60) Divisional application: 10184157.5
(73) Proprietor: Vital Health Sciences Pty Ltd., Melbourne, VIC 3000 (AU)
(72) Inventor: WEST, Simon, Michael, Williamstown, VIC 3016 (AU)
(74) Representative: Berryman, Natalia Grace
(86) International application number: PCT/AU2001/001475
(87) International publication number: WO 2002/040033

(56) References cited:
- WO-A-00/71094
- WO-A-95/31217
- US-A- 5 387 579
- US-A- 6 046 181
- J. J. BIKERMAN: J. PHYS. CHEM., vol. 56, 1952, pages 164-165, XP001203471
- DATABASE WPI Week 199807, Derwent Publications Ltd., London, GB; Class B02, AN 1998-071819, XP002968243 & JP 9 309 813 A (NONOGAWA SHOJI KK) 02 December 1997
- DATABASE CA [Online] XP002968708 Retrieved from STN Database accession no. 111:45280 & JP 63 139 972 A (SHISEIDO CO. LTD. ET AL.) 11 June 1988

## Description

The invention relates to a therapeutic emulsion formulation containing mono-electron transfer agent phosphate derivatives and di-electron transfer agent phosphate derivatives.

The invention also relates to the use of a surface active agent for increasing the surface activity and detergency of a composition.

In this specification, where a document, act or item of knowledge is referred to or discussed, this reference or discussion is not to be taken as an admission that the document, act or item of knowledge or any combination thereof was at the priority date:
(a) part of common general knowledge; or
(b) known to be relevant to an attempt to solve any problem with which this specification is concerned.

Whilst the following discussion concerns tocopherol and dermal therapy, it is also to be understood that the same principles apply to any application in which a therapeutic formulation containing electron transfer agents may be used.

EP-A-798 305 relates to a highly purified tocopheryl phosphate and/or a salt thereof wherein a P,P'-bistocopheryl diphoshate and/or a salt thereof is contained in a proportion of not higher than 3% by weight based on the weight.

A cosmetic composition for enhancing the rate of mammalian skin exfoliation comprising an effective amount of at least one ascorbic acid derivative selected from the group consisting of esters, salts, and mixtures thereof in a pharmaceutically acceptable medium for topical application is disclosed in WO 00/71094.

The skin is the largest organ of the body, and, among other things, functions to protect the internal organs from external chemical, physical and pathological hazards. Normal skin is composed of an outer epidermis covering sub dermal layers, where each layer comprises different sections. The outer cornified layer of the epidermis possesses properties of strength, flexibility, high electrical impedance and dryness that retards penetration and proliferation of microorganisms. The cornified protective layer is formed by the migration of maturing keratinocytes that are formed at the junction of the dermis and epidermis.

Vitamin E (tocopherol) is an essential part of skin dynamics and is known to be very important for skin health, with deficiency manifesting as a cornified, scaly delicate skin, thickened epidermis, scaling, lesions, chronic infection, inflammation and erythema. Vitamin E is the main naturally occurring lipid soluble agent protecting the skin from stress, and is the main lipid soluble agent protecting the cell membrane lipids from peroxidation.

Skin is subject to constant stress due to exposure to everyday elements - sun, wind and water. As a result, it is common for many topical personal care products such as lotions, moisturizers, shampoo and conditioners to contain vitamin E in various forms to assist in maintaining skin health. In order to assist in maintaining skin health, it is necessary for the vitamin E to reach the target area of the dermis. The most direct method of achieving this targeting is to apply a topical formulation to the affected area. However, topical application of vitamin E to the skin using current formulations has variable success due to the skin's ability to erect an impenetrable barrier to many outside elements. It is critical to provide for the penetration of vitamin E through the epidermis to the dermis.

The use of free tocopherol is avoided because it is unstable, therefore suitable derivatives must be found. In the alimentary canal, it has been found that there is lipase activity which releases free tocopherol from the esters of tocopherol, typically the acetate ester. This lipase activity enables the use of tocopheryl acetate as a nutritional source of Vitamin E.

In contrast, the surface of the skin is deficient in lipase activity unless it is infected with microorganisms that are able to digest sebaceous excretions. Thus tocopheryl acetate must first diffuse through the epidermis into the vital derma, where the cells have a very limited lipase activity which releases the Vitamin E. It is believed that topical formulations using tocopherol acetate have not been able to deliver adequate tocopherol beyond the epidermal layers, and therefore provide little benefit Since tocopheryl acetate is a lipidic material requiring formulation with an oil in water emulsion, absorption from such a formulation is less than optimal.

The epidermis is permeable to water soluble substances, such as tocopheryl phosphate. Until now producers of formulations containing tocopheryl phosphate utilized mono-tocopheryl phosphate isolated from the mixture produced during phosphorylation. The phosphorylation has been typically achieved using phosphorous oxychloride. The product was purified because it was believed that the by-products were deleterious to the efficacy of the mono-tocopheryl phosphate because not all the by-products were water soluble. The perceived deleterious effects were considered significant enough to justify the cost of complicated purification processes. Typically, the purification is performed by using ethanol to extract the di-tocopheryl phosphate and free tocopherol by-products.

It has been found that the use of a non-purified or semi-purified electron transfer agent phosphorylation therapeutic product is efficacious. In particular, the non-water soluble di-electron transfer agent phosphate derivatives do not have a deleterious effect on the efficacy of the therapeutic product and may even provide a synergistic effect which results in beneficial properties which enhance the dermal penetration and/or efficacy of the mono-electron transfer agent phosphate derivatives.

According to a first aspect of the invention, there is provided an emulsion composition for therapeutic administration comprising the following:
(a) at least one mono-electron transfer agent phosphate derivative;
(b) at least one di-electron transfer agent phosphate derivative;
   wherein the amount of mono-electron transfer agent phosphate derivative is no less than equimolar to the amount of di-electron transfer agent phosphate derivative wherein the electron transfer agent is selected from the group consisting of hydroxy chromans, tocols and tocotrienols in enantiomeric and raecemic forms; quinols being the reduced forms of vitamin K1 and ubiquinone; hydroxy carotenoids; and ascorbic acid; and wherein the molar ratio of mono-electron transfer agent phosphate derivative to di-electron transfer agent phosphate derivative is in the range from 85 : 15 to 65 : 35; and
(c) a suitable carrier.

According to a second aspect of the invention, there is provided the use of a surface active agent selected from the group consisting of mono-tocopheryl phosphate, a salt, or a mixture thereof for increasing the surface activity and detergency of a composition according to the invention wherein the mono-electron transfer agent phosphate derivative is mono-tocopheryl phosphate, a salt, or a mixture thereof.

The term "electron transfer agent" is used herein to refer to the class of chemicals which may be phosphorylated and which (in the non-phosphorylated form) can accept an electron to generate a relatively stable molecular radical or accept two electrons to allow the compound to participate in a reversible redox system. Electron transfer agent compounds that may be phosphorylated are hydroxy chromans including alpha-, beta- and gamma- tocopherol, tocols and tocotrienols in enantiomeric and racemic forms; quinols being the reduced forms of vitamin K1 and ubiquinone; hydroxy carotenoids including retinol; and ascorbic acid.

The phosphate derivatives of electron transfer agents comprise compounds covalently bound by means of an oxygen to the phosphorus atom of a phosphate group. The oxygen atom is typically derived from a hydroxyl group on the electron transfer agents. The phosphate derivative may exist in the form of a free phosphate acid, a salt thereof, a di-phosphate ester thereby including two molecules of electron transfer agent, a mixed ester including two different compounds selected from electron transfer agents, a phosphatidyl compound wherein the free phosphate oxygen forms a bond with an alkyl or substituted alkyl group, or a complex with a complexing agent selected from amphoteric surfactant, cationic surfactant, amino acids having nitrogen functional groups or proteins rich in these amino acids.

Examples of acceptable salts of mono-tocopherol phosphate derivatives are selected from the group consisting of the di-sodium, di-potassium, di-lithium, di-magnesium, mono-sodium, mono-potassium, mono-lithium, or mono-magnesium salts or mixtures thereof. Preferably, the acceptable salts of di-tocopheryl phosphate derivatives are selected from the sodium, potassium, lithium or magnesium salts. The di-tocopheryl phosphate derivatives will usually only form a salt in the environment required to form the di-metal salts of mono-tocopheryl phosphate derivatives.

The molar ratio of mono-electron transfer agent phosphate derivatives to di-electron transfer agent phosphate derivatives is in the range from 85:15 to 65:35. There must be enough di-electron transfer agent phosphate derivatives to form an emulsion and prevent the mono-electron transfer agent phosphate derivatives from going completely into solution, but not so much di-electron transfer agent phosphate derivatives that there is precipitation.

The mixture of mono-electron transfer agent phosphate derivatives and di-electron transfer agent phosphate derivatives can be prepared by recombining the purified individual components or by using the unpurified or semi-purified reaction product of a phosphorylation process. Preferably, the mixture is obtained by using the reaction product of a phosphorylation process. The source of a mixture of tocopheryl phosphate derivatives is preferably the reaction product of the phosphorylation of tocopherol using P₄O₁₀.

The term "acceptable carrier" is used herein to refer to a carrier considered by those skilled in the drug, food or cosmetic arts to be non-toxic when used to treat humans, animals or plants in parenteral or enteral formulations. The carrier chosen will depend on the route of administration. Ingestible formulations includes tablets, capsules, powders, chewable tablets, capsules, oral suspensions, children's formulations, enteral feeds, nutraceuticals and functional foods. For a topical application, the carrier typically comprises hydrophilic substances such as water, glycerol, polyethyleneglycol, sorbitol or propanol. For example, the composition could be used as a shampoo, hair conditioner, moisturizing cream or lotion or lipstick as a topical application.

According to a third aspect of the invention, there is a process for preparing a therapeutic formulation containing phosphate derivatives of electron transfer agents as defined above comprising the steps of:
(a) phosphorylating one or more electron transfer agents using P₄O₁₀ to form a mixture of at least one mono-electron transfer agent phosphate derivative and at least one di-electron transfer agent phosphate derivative; and
(b) combining the mixture of mono-electron transfer agent phosphate derivative and di-electron transfer agent phosphate derivative with a suitable carrier.

The mono-electron transfer agent phosphate derivatives have good water solubility, therefore before they can be absorbed into the skin or hair an aqueous topically applied composition must dry. In contrast, di-electron transfer agent phosphate derivatives are not water soluble and cause the formation of an unstable emulsion when emulsified with water and other hydrophilic solvents. Without wishing to be bound by theory, it is noted that skin is hydrophobic so when the composition is spread onto the skin, the droplets in the emulsion are attracted to the skin. The micelles become unstable near a hydrophobic surface and break so the mono-electron transfer agent phosphate derivatives are released onto the skin. The mono-electron transfer agent phosphate derivatives can then diffuse through the epidermis into the derma. Therefore, di-electron transfer agent phosphate derivatives (once considered a nuisance by-product) function as an effective spreading agent for the mono-electron transfer agent phosphate derivatives.

Again, without wishing to be bound by theory, it is considered necessary for a product which is being ingested to have several types of surface activity including detergency and appropriate surface tension to facilitate absorption. Mono-electron transfer agent phosphate derivatives may have strong detergency but do not have sufficient surface tension effects. Therefore, the mixture of mono-electron transfer agent phosphate derivatives and di-electron transfer agent phosphate derivatives having self-emulsification properties which include both types of surface activity, that is, strong detergency and strong surface tensions, will be better absorbed, especially in the small intestine.

It has surprisingly further been found that pure mono-tocopheryl phosphate and its salts are powerful surface active agents and detergents giving a stable foam.

According to a fourth aspect of the invention, there is provided the use of a surface active agent selected from the group consisting of mono-tocopheryl phosphate, its salts and mixtures thereof for increasing the surface activity and detergency of a composition according to claims 3.

Again, whilst not wishing to be bound by theory, it is thought that this detergent property may be due to the fact that mono-tocopheryl phosphate is in the form of a polar head and a non-polar tail. In contrast, di-tocopheryl phosphate has 2 non-polar tails and a polar central group which makes it surface active but it is not a detergent because at high concentrations it accumulates in the surface layer of the composition and acts as a foam breaker because the surface becomes predominantly non-polar.

### Examples

The invention will now be further illustrated and explained by reference to the following nonlimiting examples.

### Example 1

In this example, a therapeutic formulation according to the invention was prepared using tocopherol as the electron transfer agent

### Preparation of the tocopheryl phosphate mixture

500 g dl-alpha-tocopherol was taken and mixed with a high shear mixer with 4 aliquots each of 21g of P₄O₁₀ at 12 minute intervals, holding the temperature above 60°C. While the mixture was still hot, 91.5 g of sodium hydroxide which had been dissolved in 62.5 g of water at 50°C were added over 1.5 hours to hydrolyse and neutralise the tocopheryl phosphates. The product was cooled to ambient temperature then further cooled with liquid nitrogen to give a brittle product that was ground to a powder and dried under vacuum.

The mole ratio of mono-tocopheryl phosphate to di-tocopheryl phosphate was approximately 70:30. The product contained mono- and di-sodium tocopheryl phosphate (approx. 65-85% by mole), sodium di-tocopheryl phosphate (approx. 10-35% by mole) and some sodium di-tocopheryl pyrophosphate.

### Reparation and application of the topical formulation

The dried powder was dispersed in water as a 5% solution. 10 ml of this solution was applied to the hands to give a satisfactory application of the tocopheryl phosphates to the skin.

### Example 2

The skin penetration properties of a mixture of mono- and di-tocopheryl phosphates according to the invention were compared to tocopheryl acetate.

### Test formulations

The test materials were made up on the basis of 5% mixed actives (mono-tocopheryl phosphate (TP), di-tocopheryl phosphate (T2P) or tocopheryl acetate) in a vehicle consisting of 95/5 distilled water/ethanol with pH adjusted (if necessary to 6.5-7.0 with citric acid or dilute NaOH).

### TP and T2P (mixed sodium salts)

A slurry of 6.25 w/w % of 80% mixed TP and T2P in 93.75 w/w % of the 95/5-water/ethanol mixture was prepared.

| Active | TP & T2P (micrograms per applied dose) |
|---|---|
| tocopheryl phosphate | 252 |
| di-tocopheryl phosphate | 1194 |
| tocopherol | 24 |

### TP and T2P complexed

The TPC used was lauryl-imino di-propionic acid tocopheryl phosphate, a surface-active amphoteric phosphate ester complex formed from lauryl imino propionic acid (Deriphat 160) and tocopheryl phosphates. The solution was based on 40% active mixed phosphates as the latter was reacted/combined in a 60/40-amphoteric/mixed-phosphate weight ratio (1.9-1 mole ratio). 12.5 w/w % of the complex was dissolved in 87.5 w/w % of 95/5 water/ethanol mixture.

| Active | TP and T2P complexed (micrograms per applied dose) |
|---|---|
| tocopheryl phosphate | 188 |
| di-tocopheryl phosphate | 713 |
| Tocopherol | 20 |

### Tocopheryl Acetate

Tocopheryl acetate was obtained from Roche/BASF. 5.0 w/w % of tocopheryl acetate was dispersed in 95.0 w/w % of 95/5 water/ethanol mixture.

### Method

The test formulations are evaluated in *in vitro* human skin penetration studies. Samples are analyzed for the mono- and di-tocopheryl phosphates, free alpha-tocopherol, and tocopheryl acetate by high performance liquid chromatography (HPLC). The tests are conducted by DermTech International (San Diego, CA). Human cadaver skin samples are obtained and prepared. Each formulation is evaluated on triplicate sections from each donor at a topically applied dose of 5 µL/cm². Receptor solutions are collected over 48 hours at pre-selected time intervals. After 48 hours the skin surface is washed with isopropyl alcohol, and the skin is collected and split into epidermis and dermis. The skin sections are extracted with isopropyl alcohol. All collected samples are processed and assayed for tocopherol, tocopheryl acetate, tocopheryl phosphate and di-tocopheryl phosphate.

Mass balance from the samples is between 80-120% of the applied dose.

No tocopherols are observed in the receptor solution. This could be a result of amounts being below limits of detection, or degradation of the various tocopherol species into other, as yet uncharacterized, compounds.

**TABLE 1: SKIN PENETRATION STUDY**

| (Percent Distribution of Tocopherols Recovered; wt/wt %). | | | |
|---|---|---|---|
| **Treatment** | **α-Tocopherol** | **TP** | **T₂P** |
| **TP & T2P (mixed sodium salts)** | | | |
| Surface wash | 65.05 | 41.40 | 56.05 |
| Epidermis | 26.74 | 47.06 | 37.31 |
| Dermis | 8.24 | 11.42 | 6.62 |
| Dermis/Epidermis Ratio | 0.31 | 0.24 | 0.18 |
| | | | |

| **TP & T2P complexed** | | | |
|---|---|---|---|
| Surface wash | 50.00 | 48.82 | 70.92 |
| Epidermis | 35.99 | 24.55 | 16.67 |
| Dermis | 14.07 | 26.62 | 12.36 |
| Dermis/Epidermis Ratio | 0.39 | 1.08 | 0.74 |
| | | | |

| **Tocopherol Acetate** | **α-Tocopherol** | - | - |
|---|---|---|---|
| Surface wash | 91.48 | | |
| Epidermis | 7.13 | | |
| Dermis | 1.39 | | |
| Dermis/Epidermis Ratio | 0.20 | | |

### Conclusions

The results demonstrate that the inclusion of 20 to 30% of T2P in the formulation did not have a deleterious effect on the performance of the tocopheryl phosphate product. Further, both of the TP/T2P mixtures were more efficiently transported into the dermis than the tocopheryl acetate product.

### Example 3

In this example, a mixture was prepared comprising mono-ubiquinyl phosphate and di-ubiquinyl phosphate made according to the invention.

100g of ubiquinone was partially dissolved in 200ml of hot glacial acetic acid. To the vigorously stirred solution, small amounts of zinc (total of 30g) were added until the solution changed from yellow to green and then became colorless. The hot solution was filtered and the unreacted zinc was washed 2 more times (50ml) with hot glacial acetic acid to recover any retaining ubiquinol. Glacial acetic acid was removed from the ubiquinol by vacuum distillation or by cooling the solution to 0°C and filtering off the crystallized ubiquinol. To further remove any traces of acetic acid, the ubiquinol was placed under high vacuum (1mm Hg) for a period of 2 hours.

The ubiquinol product was treated immediately by heating to 100°C and adding 33g of P₄O₁₀. The mixture was stirred for 3 hours and then 500 ml water was introduced slowly into the mixture. The temperature of the reaction was maintained just below boiling point for a further 1 hour. Removal of water yielded ubiquinyl phosphates and phosphoric acid. The phosphoric acid was partially removed by further washes with hot water.

The final product consisted of 139g of mono-ubiquinyl phosphate, di-ubiquinyl phosphate and phosphoric acid. The product was analyzed by ³¹P NMR and the molar ratio of mono-ubiquinyl phosphate : di-ubiquinyl phosphate was 76:24.

### Example 4

In this example, the surface active properties of mono-tocopheryl phosphate were investigated. 0.1 g of pure di-sodium mono-tocopheryl phosphate was dissolved in 10 ml of pure distilled water in a 50 ml cylindrical stoppered vessel. The vessel was shaken on a test tube agitator and the headspace filled with stable foam. The foam was examined on a daily basis and showed complete stability for one day and then slowly degraded over the rest of the four-day period.

Mono-tocopheryl phosphate is therefore a surface active agent with detergent properties.

The word 'comprising' and forms of the word 'comprising' as used in this description and in the claims does not limit the invention claimed to exclude any variants or additions.

## Claims

1. An emulsion composition for therapeutic administration comprising:
(a) at least one mono-electron transfer agent phosphate derivative;
(b) at least one di-electron transfer agent phosphate derivative;
wherein the electron transfer agent is selected from the group consisting of hydroxy chromans, tocols and tocotrienols in enantiomeric and raecemic forms; quinols being the reduced forms of vitamin Kl and ubiquinone; hydroxy carotenoids; and ascorbic acid; and
wherein the molar ratio of mono-electron transfer agent phosphate derivative to di-electron transfer agent phosphate derivative is in the range from 85 : 15 to 65 : 35; and
(c) a suitable carrier.

2. The composition according to claim 1, wherein the mono-electron transfer agent phosphate derivative and the di-electron transfer agent phosphate derivative are derived from alpha-tocopherol.

3. The composition according to claim 1, wherein the mono-electron transfer agent phosphate derivative is mono-tocopheryl phosphate, a salt, or a mixture thereof.

4. The composition according to claim 1, wherein the electron transfer agent is tocopherol.

5. The composition according to any of claims 1 to 4, wherein the phosphate derivative of the electron transfer agent exists in the form of a free phosphate acid, a salt thereof, a di-phosphate ester including two molecules of electron transfer agent, a mixed ester including two different electron transfer agents, or a phosphatidyl compound wherein the free phosphate oxygen forms a bond with an alkyl or substituted alkyl group.

6. The composition according to claim 5, wherein the phosphate derivative of the electron transfer agent is a salt of mono-tocopherol phosphate which is selected from the group consisting of di-sodium, di-potassium, di-lithium, di-magnesium, mono-sodium, mono-potassium, mono-lithium, and mono-magnesium salts or mixtures thereof.

7. The composition according to claim 5, wherein the phosphate derivative of the electron transfer agent is a salt of di-tocopheryl phosphate which is selected from the group consisting of sodium, potassium, lithium, and magnesium salts.

8. The composition according to any of claims 1 to 7, wherein the mono-electron transfer agent phosphate derivative and the di-electron transfer agent phosphate derivative are complexed with a complexing reagent selected from amphoteric surfactants, cationic surfactants, amino acids having nitrogen functional groups and proteins rich in these amino acids.

9. The composition according to any of claims 1 to 8 in a form of an ingestible formulation selected from the group consisting of capsules, children's formulations, enteral feeds, nutraceuticals and functional foods.

10. The composition according to any of claims 1 to 8 in a form suitable for topical application.

11. The composition according to claim 10 in the form of a shampoo, hair conditioner, moisturizing cream, or lotion.

12. A process for preparing a therapeutic composition according to any of claims 1 to 11 comprising the steps of:
(d) phosphorylating one or more electron transfer agents using P₄O₁₀ to form a mixture of at least one mono-electron transfer agent phosphate derivative and at least one di-electron transfer agent phosphate derivative; and
(e) combining the mixture of mono-electron transfer agent phosphate derivative and di-electron transfer agent phosphate derivative with a suitable carrier.

13. The process according to claim 12, wherein the electron transfer agent is tocopherol.

14. Use of a surface active agent selected from the group consisting of mono-tocopheryl phosphate, a salt, or a mixture thereof for increasing the surface activity and detergency of a composition as defined in claim 3.

## Patentansprüche

1. Eine Emulsionszusammensetzung zur therapeutischen Verabreichung, umfassend:
(a) mindestens ein Phosphatderivat eines Einelektronentransfermittels;
(b) mindestens ein Phosphatderivat eines Dielektronentransfermittels;
wobei das Elektronentransfermittel aus der aus Hydroxychromanen, Tocolen und Tocotrienolen in enantiomeren und racemischen Formen; Chinolen, welche die reduzierten Formen von Vitamin K1 und Ubichinon sind; Hydroxycarotenoiden; und Ascorbinsäure bestehenden Gruppe ausgewählt ist; und
wobei das Molverhältnis des Phosphatderivats eines Einelektronentransfermittels zu dem Phosphatderivat eines Dielektronentransfermittels in dem Bereich von 85 : 15 bis 65 : 35 ist; und
(c) einen geeigneten Träger.

2. Die Zusammensetzung gemäß Anspruch 1, wobei das Phosphatderivat eines Einelektronentransfermittels und das Phosphatderivat eines Dielektronentransfermittels sich von alpha-Tocopherol ableiten.

3. Die Zusammensetzung gemäß Anspruch 1, wobei das Phosphatderivat eines Einelektronentransfermittels mono-Tocopherylphosphat, ein Salz oder ein Gemisch davon ist.

4. Die Zusammensetzung gemäß Anspruch 1, wobei das Elektronentransfermittel Tocopherol ist.

5. Die Zusammensetzung gemäß einem der Ansprüche 1 bis 4, wobei das Phosphatderivat des Elektronentransfermittels in Form einer freien Phosphorsäure, eines Salzes davon, eines Diphosphatesters, der zwei Moleküle eines Elektronentransfermittels enthält, eines gemischten Esters, der zwei verschiedene Elektronentransfermittel enthält, oder einer Phosphatidylverbindung, wobei das freie Sauerstoffatom des Phosphats eine Bindung mit einem Alkyl- oder einem substituierten Alkylrest bildet, vorhanden ist.

6. Die Zusammensetzung gemäß Anspruch 5, wobei das Phosphatderivat des Elektronentransfermittels ein Salz eines mono-Tocopherolphosphats ist, welches aus der aus Dinatrium-, Dikalium-, Dilithium-, Dimagnesium-, Mononatrium-, Monokalium-, Monolithium- und Monomagnesium-Salzen oder Gemischen davon bestehenden Gruppe ausgewählt ist.

7. Die Zusammensetzung gemäß Anspruch 5, wobei das Phosphatderivat des Elektronentransfermittels ein Salz eines di-Tocopherylphosphats ist, welches aus der aus Natrium-, Kalium-, Lithium- und Magnesium-Salzen bestehenden Gruppe ausgewählt ist.

8. Die Zusammensetzung gemäß einem der Ansprüche 1 bis 7, wobei das Phosphatderivat eines Einelektronentransfermittels und das Phosphatderivat eines Dielektronentransfermittels mit einem aus amphoteren grenzflächenaktiven Mitteln, kationischen grenzflächenaktiven Mitteln, Aminosäuren, die funktionelle Stickstoffreste aufweisen, und Proteinen, die reich an diesen Aminosäuren sind, ausgewählten Komplexbildner komplexiert sind.

9. Die Zusammensetzung gemäß einem der Ansprüche 1 bis 8 in Form einer einnehmbaren Formulierung, ausgewählt aus der aus Kapseln, Formulierungen für Kinder, enteraler Ernährung, Nutrazeutika und funktionellen Lebensmitteln bestehenden Gruppe.

10. Die Zusammensetzung gemäß einem der Ansprüche 1 bis 8 in einer zur topischen Anwendung geeigneten Form.

11. Die Zusammensetzung gemäß Anspruch 10 in Form eines Shampoos, einer Haarspülung, einer Feuchtigkeitscreme oder einer Lotion.

12. Ein Verfahren zur Herstellung einer therapeutischen Zusammensetzung gemäß einem der Ansprüche 1 bis 11, umfassend die Schritte:
(d) Phosphorylieren eines oder mehrerer Elektronentransfermittel unter Verwenden von P₄O₁₀, um ein Gemisch von mindestens einem Phosphatderivat eines Einelektronentransfermittels und mindestens einem Phosphatderivat eines Dielektronentransfermittels zu bilden; und
(e) Kombinieren des Gemischs des Phosphatderivats eines Einelektronentransfermittels und des Phosphatderivats eines Dielektronentransfermittels mit einem geeigneten Träger.

13. Das Verfahren gemäß Anspruch 12, wobei das Elektronentransfermittel Tocopherol ist.

14. Verwendung eines grenzflächenaktiven Mittels, ausgewählt aus der aus mono-Tocopherylphosphat, einem Salz oder einem Gemisch davon bestehenden Gruppe, zur Erhöhung der Grenzflächenaktivität und Waschkraft einer wie in Anspruch 3 definierten Zusammensetzung.

## Revendications

1. Composition d'émulsion pour l'administration thérapeutique comprenant :
(a) au moins un dérivé de phosphate de mono-agent de transfert d'électrons ;
(b) au moins un dérivé de phosphate de di-agent de transfert d'électrons ;
où l'agent de transfert d'électrons est choisi dans le groupe consistant en les hydroxychromanes, les toco!s et les tocotriénols dans des formes énantiomères et racémiques ; les quinols qui sont les formes réduites de la vitamine K1 et de l'ubiquinone ; les hydroxycaroténoïdes ; et l'acide ascorbique ; et
où le rapport molaire du dérivé de phosphate de mono-agent de transfert d'électrons au dérivé de phosphate de di-agent de transfert d'électrons est dans la plage de 85:15 à 65:35 ; et
(c) un vecteur approprié.

2. Composition selon la revendication 1 où le dérivé de phosphate de mono-agent de transfert d'électrons et le dérivé de phosphate de di-agent de transfert d'électrons sont dérivés de l'alphatocophérol.

3. Composition selon la revendication 1 où le dérivé de phosphate de mono-agent de transfert d'électrons est le phosphate de mono-tocophéryle, un sel ou un mélange de ceux-ci.

4. Composition selon la revendication 1 où l'agent de transfert d'électrons est le tocophérol.

5. Composition selon l'une quelconque des revendications 1 à 4 où le dérivé de phosphate de l'agent de transfert d'électrons existe sous forme d'un acide de phosphate libre, d'un sel de celui-ci, d'un ester di-phosphate incluant deux molécules d'agent de transfert d'électrons, d'un ester mixte incluant deux agents de transfert d'électrons différents, où d'un composé phosphatidyle où l'oxygène de phosphate libre forme une liaison avec un groupe alkyle ou alkyle substitué.

6. Composition selon la revendication 5 où le dérivé de phosphate de l'agent de transfert d'électrons est un sel de phosphate de mono-tocophérol qui est choisi dans le groupe consistant en les sels de di-sodium, di-potassium, di-lithium, di-magnésium, mono-sodium, mono-potassium, mono-lithium et mono-magnésium ou leurs mélanges.

7. Composition selon la revendication 5 où le dérivé de phosphate de l'agent de transfert d'électrons est un sel de phosphate de di-tocophéryle qui est choisi dans le groupe consistant en les sels de sodium, de potassium, de lithium et de magnésium.

8. Composition selon l'une quelconque des revendications 1 à 7 où le dérivé de phosphate de mono-agent de transfert d'électrons et le dérivé de phosphate de di-agent de transfert d'électrons sont complexés avec un réactif complexant choisi parmi les tensioactifs amphotères, les tensioactifs cationiques, les aminoacides ayant des groupes fonctionnels azotés et les protéines riches en ces aminoacides.

9. Composition selon l'une quelconque des revendications 1 à 8 sous forme d'une formulation ingérable choisie dans le groupe consistant en les capsules, les formulations pour les enfants, les aliments entéraux, les nutraceutiques et les aliments fonctionnels.

10. Composition selon l'une quelconque des revendications 1 à 8 sous une forme appropriée pour l'application topique.

11. Composition selon la revendication 10 sous forme d'un shampoing, d'une lotion capillaire, d'une crème humectante ou d'une lotion.

12. Procédé pour préparer une composition thérapeutique selon l'une quelconque des revendications 1 à 11 comprenant les étapes de :
(d) phosphorylation d'un ou plusieurs agents de transfert d'électrons avec P₄O₁₀ pour former un mélange d'au moins un dérivé de phosphate de mono-agent de transfert d'électrons et d'au moins un dérivé de phosphate de di-agent de transfert d'électrons ; et
(e) combinaison du mélange de dérivé de phosphate de mono-agent de transfert d'électrons et de dérivé de phosphate de di-agent de transfert d'électrons avec un vecteur approprié.

13. Procédé selon la revendication 12 où l'agent de transfert d'électrons est le tocophérol.

14. Utilisation d'un agent tensioactif choisi dans le groupe consistant en le phosphate de mono-tocophéryle, un sel ou un mélange de ceux-ci pour augmenter l'activité superficielle et la détergence d'une composition selon la revendication 3.
